# EUROPÄISCHE PATENTSCHRIFT

(11) **EP 0 489 037 B1**
(45) Veröffentlichungstag und Bekanntmachung des Hinweises auf die Patenterteilung: **02.08.1995**
(21) Anmeldenummer: 90912007.3
(22) Anmeldetag: 16.08.1990
(51) Int. Cl.: A61K 7/16, A61K 7/22

(54) **BELAGHEMMENDE ZAHNPASTA**
ANTI-PLAQUE TOOTHPASTE
DENTIFRICE ANTI-PLAQUE DENTAIRE

(30) Priorität: 24.08.1989 DE 3927982
(43) Veröffentlichungstag der Anmeldung: 10.06.1992
(73) Patentinhaber: Henkel Kommanditgesellschaft auf Aktien, 40191 Düsseldorf (DE)
(72) Erfinder: WÜLKNITZ, Peter, D-4018 Langenfeld (DE); LEHMANN, Rudolf, D-5653 Leichlingen 1 (DE); KLÜPPEL, Hans-Jürgen, D-4000 Düsseldorf 30 (DE)
(86) Internationale Anmeldenummer: EP9001345
(87) Internationale Veröffentlichungsnummer: WO9102511

(56) Entgegenhaltungen:
- AT-B- 320 159
- AT-B- 337 904
- DE-A- 2 240 352
- FR-A- 2 341 302
- US-A- 4 241 049

## Beschreibung

Die Erfindung betrifft eine Zahnpaste in Form einer Dispersion von Calciumcarbonat als Poliermittel in einem wäßrigen Träger, die als plaquehemmende Komponente eine antimikrobiell wirksame Biguanidverbindung enthält und deren übrige Komponenten nach Art und Menge so ausgewählt sind, daß trotz relativ niedriger Dosierung der antimikrobiell wirksamen Biguanidverbindung eine optimale Hemmung der Zahnbelagsbildung erreicht wird.

Es ist seit langem bekannt, daß antimikrobiell wirksame Biguanidverbindungen eine die Bildung von Zahnbelägen verhindernde Wirkung haben. Diese Wirkung wird jedoch durch viele in Zahnpasten übliche Komponenten, insbesondere durch bestimmte Poliermittel, wie z. B. Kreide, aber auch durch viele Bindemittel bzw. Konsistenzregler, oberflächenaktive Stoffe, ja sogar durch bestimmte Süßungsmittel stark verringert oder ganz aufgehoben. Es hat daher nicht an Versuchen gefehlt, solche Komponenten zu finden, welche die antimikrobiellen Biguanide nicht beeinträchtigen oder diese Beeinträchtigung verhindern. So wird z. B. in DE-OS-21 58 149 vorgeschlagen, als Poliermittel α-Aluminiumoxid-trihydrat einer bestimmten Partikelgröße einzusetzen. In DE-OS-34 44 958 wird andererseits offenbart, daß bestimmte Tenside einen die Wirkung des antimikrobiellen Biguanids verstärkenden Effekt zeigen. Aber auch dieser Effekt wird in Gegenwart von Kreide als Poliermittel oder in Gegenwart von anionischen Bindemitteln bzw. Konsistenzreglern, an ionischen oberflächenaktiven Mitteln und Lösungsvermittlern gestört.

Aus FR-A-2 341 302 waren Zahnpasten mit einem Gehalt an Chlorhexidin, jeodch ohne kationische Tenside, bekannt.

Eine befriedigende Zahnpaste, die auch noch bei niedriger Dosierung der antimikrobiell wirksamen Biguanidverbindungen eine gute Hemmwirkung der Zahnbelagsbildung aufweist, ist dem Stande der Technik daher nicht zu entnehmen. Die vorliegende Erfindung hat die Lösung dieser Aufgabe für solche Zahnpasten zum Ziel, die als Poliermittel Kreide enthalten. Gegenstand der Erfindung ist eine Zahnpaste in Form einer wäßrigen Dispersion enthaltend 10 - 60 Gew.-% Poliermittel, 2 - 20 Gew.-% Feuchthaltemittel, 0,5 - 5 Gew.-% wasserlösliche Konsistenzregler, 0,05 - 0,5 Gew.-% antimikrobielle Biguanide und 1 - 5 Gew.-% weitere Zusatzstoffe aus der Gruppe der oberflächenaktiven Stoffe, der Aromaöle und Süßungsmittel, dadurch gekennzeichnet, daß als Poliermittel überwiegend Calciumcarbonat (Kreide), als Konsistenzregler nichtionische Polysaccharidderivate und als oberflächenaktive Stoffe ein kationisches Tensid mit einer linearen Alkylgruppe mit 12 - 18 C-Atomen und einer oder zwei tertiären Aminogruppen oder quartären Ammoniumgruppen und gegebenenfalls ein nichtionogener Lösungsvermittler für das Aromaöl enthalten sind.

Als Poliermittel wird bevorzugt eine gefällte Kreide gewählt, deren Partikel im wesentlichen weniger als 20 »m Durchmesser, besser noch weniger als 10 »m, bevorzugt zwischen 1 und 5 »m aufweisen.

Als Feuchthaltemittel kommen Glycerin, Sorbit, Propylenglykol und Polyethylenglykole in Frage, bevorzugt werden Glycerin und/oder Sorbit verwendet. Geeignete wasserlösliche Konsistenzregler sind die nichtionischen Polysaccharidderivate, z. B. Methyl-, Hydroxyethyl- und Hydroxypropylether von Cellulose, Stärke, Guar, Xanthan und Pflanzengummen. Bevorzugt werden Hydroxyethylcellulose und Methylhydroxypropylcellulose eingesetzt.

Als antimikrobiell wirksame Biguanidverbindung wird bevorzugt das aus GB-A-705 838 bekannte 1,1'-Hexamethylenbis-[5-(4-chlorphenyl)-biguanid] ("Chlorhexidin") in Form eines wasserlöslichen, physiologisch verträglichen Salzes, z. B. in Form des Acetats oder als Gluconat eingesetzt. Andere erfindungsgemäß geeignete antimikrobielle Biguanidverbindungen sind z. B. das 1,1'-Hexamethylenbis-[5-(4-fluorphenyl)-biguanid] ("Fluorhexidin") die aus GB-A-702 268 bekannten Polyhexamethylen-Biguanidverbindungen des Typs Vantocil IB (ICI), ferner, die aus US-A-2 684 924, US-A-2 990 425, US-A-3 468 898, US-A-4 022 834, US-A-4 053 636 und US-A-4 198 392 bekannten antimikrobiellen Biguanidverbindungen.

Als kationische Tenside sind in der erfindungsgemäßen Zahnpaste bevorzugt solche der allgemeinen Formel I 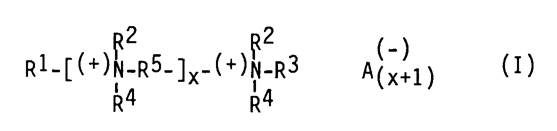
worin R¹ eine Alkyl- oder Hydroxyalkylgruppe mit 12 - 18 C-Atomen, R² und R³ eine Alkylgruppe mit 1 - 4 C-Atomen oder eine 2-Hydroxyalkylgruppe mit 2 - 4 C-Atomen, R⁴ Wasserstoff, eine Alkylgruppe mit 1 - 4 C-Atomen, eine Benzylgruppe oder eine 2-Hydroxyalkylgruppe mit 2 - 4 C-Atomen, R⁵ eine Alkylengruppe mit 1 - 4 C-Atomen, x = 0 oder 1 und A ein Fluorid-, Chlorid-, Bromid-, Methoxysulfat- oder Ethoxysulfat-Anion ist, in einer Menge von 0,01 - 1 Gew.-% enthalten. Bevorzugt geeignete kationische Tenside der Formel I sind z. B.
Cetyltrimethylammonium-chlorid
Lauryl-dimethyl-benzyl-ammoniumchlorid
Stearyl-dimethyl-benzyl-ammoniumchlorid
2-Hydroxydodecyl-2-hydroxyethyl-dimethyl-ammoniumchlorid
N-Octadecyl-N,N',N'-tris-(2-hydroxyethyl)-1,3-diamino-propan-dihydrochlorid (Aminfluorid).

Durch den Zusatz der kationischen Tenside gemäß der allgemeinen Formel I zu der erfindungsgemäßen Zahnpaste wird die Sorption der antimikrobiellen, belagverhindernden Biguanidverbindungen an dem Poliermittel merklich verringert, so daß die Biguanidverbindung in der Zahnpaste frei verfügbar bleibt und für die Belagverhinderung voll verfügbar ist. Durch den erfindungsgemäßen Zusatz der kationischen Tenside wird es möglich, belaghemmende Zahnpasten mit Calciumcarbonat als Putzkörper herzustellen. Die Erhöhung des frei verfügbaren Chlorhexidins in einer Kreidedispersion mit 0,1 Gew.-% Chlorhexidin durch Zusatz der kationischen Tenside wird in Beispiel 1 demonstriert.

Die erfindungsgemäße Zahnpaste kann durch Zugabe Von Aromaölen und Süßungsmitteln in ihren organoleptischen Eigenschaften verbessert werden. Als Aromaöle kommen alle für Hund- und Zahnpflegemittel gebräuchlichen natürlichen und synthetischen Aromen in Frage. Natürliche Aromen können sowohl in Form der aus den Drogen isolierten etherischen Ölen als -auch der aus diesen isolierten Einzelkomponenten verwendet werden. Bevorzugt sollte wenigstens ein Aromaöl aus der Gruppe Pfefferminzöl, Krauseminzöl, Anisöl, Sternanisöl, Kümmelöl, Eukalyptusöl, Fenchelöl, Zimtöl, Nelkenöl, Geraniumöl, Salbeiöl, Pimentöl, Thymianöl, Majoranöl, Basilikumöl, Citrusöl, Gaultheriaöl oder eine oder mehrere daraus isolierte oder synthetisch erzeugte Komponenten dieser Öle enthalten sein. Die wichtigsten Komponenten der genannten Öle sind z. B. Menthol, Carvon, Anethol, Cineol, Eugenol, Zimtaldehyd, Caryophyllen, Geraniol, Citronellol, Linalool, Salven, Thymol, Terpinen, Terpinol, Methylchavicol und Methylsalicylat. Weitere geeignete Aromen sind z. B. Menthylacetat, Vanillin, Jonone, Linalylacetat, Rhodinol und Piperiton.

Zur Solubilisierung dieser meist wasserunlöslichen Aromaöle in der Zahnpaste ist erfindungsgemäß ein nichtionogener Lösungsvermittler erforderlich. Solche Lösungsvermittler gehören zur Gruppe der oberflächenaktiven Verbindungen. Ein weiterer Gegenstand der Erfindung ist daher eine erfindungsgemäße Zahnpaste, die 0,1 - 0,5 Gew.-% eines Aromaöls und 0,1 - 0,7 Gew.-% eines nichtionogenen Lösungsvermittlers, bevorzugt aus der Gruppe der oxethylierten Fettsäureglyceride, der oxethylierten Fettsäure-sorbitanpartialester oder der Fettsäurepartialester von Glycerin- oder Sorbitanoxethylaten enthält.

Lösungsvermittler aus der Gruppe der oxethylierten Fettsäureglyceride umfassen vor allem Anlagerungsprodukte von etwa 20 - 60 Mol Ethylenoxid an Mono- und Diglyceride von linearen Fettsäuren mit 12 - 18 C-Atomen oder an Triglyceride von Hydroxyfettsäuren wie Oxystearinsäure oder Ricinolsäure. Weitere geeignete Lösungsvermittler sind oxethylierte Fettsäuresorbitanpartialester - das sind bevorzugt Anlagerungsprodukte von 20 - 60 Mol Ethylenoxid an Sorbitanmonoester und Sorbitandiester von Fettsäuren mit 12 - 18 C-Atomen. Ebenfalls geeignete Lösungsvermittler sind Fettsäurepartialester von Glycerin- oder Sorbitan-oxethylaten; das sind bevorzugt Mono- und Diester von C₁₂-C₁₈-Fettsäuren und Anlagerungsprodukten von 20 - 60 Mol Ethylenoxid an 1 Mol Glycerin oder 1 Mol Sorbit.

Die erfindungsgemäße Zahnpaste enthält bevorzugt als Lösungsvermittler für gegebenenfalls enthaltene Aromaöle Anlagerungsprodukte von 20 - 60 Mol Ethylenoxid an gehärtetes oder ungehärtetes Ricinusöl (d. h. an Oxystearinsäure- oder Ricinolsäure-triglycerid), an Glycerinmono- und/oder -distearat oder an Sorbitanmono- und/oder -distearat.

Als Süßungsmittel eignen sich entweder natürliche Zucker, z. B. Sucrose, Maltose, Lactose und Fructose oder synthetische Süßstoffe, jedoch bevorzugt nichtionogene oder amphotere Stoffe. Bevorzugt geeignet als Süßungsmittel ist der L-Aspartyl-L-phenyl-alanin-methylester, im Handel unter der Warenbezeichnung Aspartame^{(R)}.

Es können auch weitere bekannte Zahnpastenzusätze in untergeordneten Mengen von insgesamt bis zu höchstens 3 Gew.-% zugegeben werden, soweit diese mit dem antimikrobiellen Biguanid verträglich sind und dessen Wirkung nicht beeinträchtigen. Solche Zusätze sind z. B.
- karieshemmende Stoffe wie Natriumfluorid oder Natrium-monofluorphosphat,
- Pigmente wie z. B. Titandioxid,
- Farbstoffe
- pH-Stellmittel und Puffersubstanzen, z. B. Citronensäure und deren Salze oder Phosphorsäure und deren Alkalisalze
- wundheilende und entzündungshemmende Stoffe wie z. B. Allantoin, Harnstoff, Azulen bzw. Kamillenwirkstoffe.

Die folgenden Beispiele sollen den Erfindungsgegenstand näher erläutern ohne ihn hierauf zu beschränken.

### Beispiele

1. Prüfung der Steigerung des Gehalts an frei verfügbarem analytisch nachweisbarem Chlorhexidin nach Zusatz von kationischem Tensid.
Es wurde eine Standard-Dispersion von 40 Gew. % Kreide (Calciumcarbonat) und 0,1 Gew.-% Chlorhexidin in Wasser hergestellt und mit steigenden Mengen an kationischen Tensiden versetzt. Nach Zusatz des kationischen Tensids wurden die Dispersionen 24 Stunden zur Einstellung der Gleichgewichtslage bei 20 °C geschüttelt. Dann wurden die Dispersionen mit 3 Gewichtsteilen Wasser pro Gewichtsteil Dispersion verdünnt, vom Calciumcarbonat abfiltriert und im klaren Filtrat auf den Gehalt an freiem Chlorhexidin geprüft. Die analytische Bestimmung des freien Chlorhexidin erfolgte nach der von Cropper, Platt und Puttnam in J. Soc. Cosmet. Chem. 26, (1975), Seite 355 - 373 angegebenen Methode.
Es wurden die folgenden kationischen Tenside verwendet:
- A:: Cetyltrimethylammonium-bromid
- B:: 2-Hydroxydodecyl-2-hydroxyethyl-dimethyl-ammoniumchlorid
- C:: N-Octadecyl-N,N'N'-tris-(2-hydroxyethyl)-1,3-diaminopropan-dihydrofluorid (sog. Aminfluorid).
Die Art und Konzentration des zugesetzten kationischen Tensids und die Menge an frei verfügbarem Chlorhexidin nach 24stündiger Gleichgewichtseinstellung in % der zugesetzten Menge, ist der folgenden Tabelle zu entnehmen.

| Versuch Nr. | 1 | 2 | 3 | 4 | 5 | 6 | 7 | 8 | 9 | 10 | 11 | 12 |
|---|---|---|---|---|---|---|---|---|---|---|---|---|
| kation. Tensid | - | A | A | A | A | B | B | B | B | C | C | C |
| Konzentration Gew.-% | - | 0,01 | 0,05 | 0,1 | 0,5 | 0,01 | 0,05 | 0,1 | 0,5 | 0,17 | 0,33 | 0,66 |
| freies Chlorhexidin in % | 15 | 15 | 27 | 64 | 90 | 20 | 20 | 20 | 33 | 20 | 20 | 85 |

2. Erfindungsgemäße Zahnpaste

| Rezeptur: | |
|---|---|
| Kreide (gefälltes Calciumcarbonat) | 40,0 Gew.-% |
| Sorbit (70 %ige Lösung) | 5,0 Gew.-% |
| Glycerin | 5,0 Gew.-% |
| Methyl-hydroxypropylcellulose¹⁾ | 2,5 Gew.-% |
| Hydroxyethylcellulose²⁾ | 1,3 Gew.-% |
| kation. Tensid C (Aminfluorid) | 1,0 Gew.-% |
| Pfefferminz-Aromaöl | 0,3 Gew.-% |
| HR 60³⁾ | 0,3 Gew.-% |
| Chlorhexidin-digluconat | 0,2 Gew.-% |
| Aspartame^{(R)} | 0,01 Gew.-% |
| Wasser | ad 100 Gew.-% |

| | |
|---|---|
| ¹⁾ Es wurde das Handelsprodukt Culminal MHPC 100 (Henkel KGaA) eingesetzt. | |
| ²⁾ Es wurde das Handelsprodukt Cellobond HEC (BP) eingesetzt. | |
| ³⁾ Anlagerungsprodukt von 60 Mol Ethylenoxid an gehärtetes Ricinusöl. | |

## Patentansprüche

1. Zahnpaste in Form einer wäßrigen Dispersion, enthaltend
10,0 - 60,0 Gew.-% Poliermittel,
2,0 - 20,0 Gew.-% Feuchthaltemittel,
0,5 - 5,0 Gew.-% wasserlösliche Konsistenzregler,
0,05- 0,5 Gew.-% antimikrobielle Biguanide und
1,0 - 5,0 Gew.-% weiterer Zusatzstoffe aus der Gruppe der ober
flächenaktive Stoffe, der Aromaöle und Süßungsmittel, dadurch gekennzeichnet, daß
- als Poliermittel überwiegend Calciumcarbonat
- als Konsistenzregler nichtionische Polysaccharidderivate und
- als oberflächenaktive Stoffe ein kationisches Tensid mit einer linearen Alkylgruppe mit 12 - 18 C-Atomen und einer oder zwei tertiären Aminogruppen oder quartären Ammoniumgruppen und gegebenenfalls ein nichtionogener Lösungsvermittler für das Aromaöl enthalten sind.

2. Zahnpaste gemäß Patentanspruch 1, dadurch gekennzeichnet, daß als antimikrobielles Biguanid das 1,1'-Hexamethylen-bis-(4-chlorphenyl)-biguanid (Chlorhexidin) in Form eines wasserlöslichen Salzes enthalten ist.

3. Zahnpaste nach einem der Ansprüche 1 oder 2, dadurch gekennzeichnet, daß als kationisches Tenside ein solches der Formel I 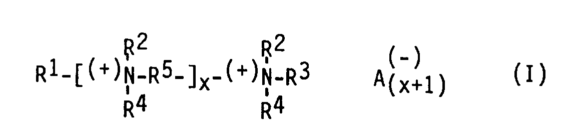 in der R¹ eine Alkyl- oder Hydroxyalkylgruppe mit 12 - 18 C-Atomen, R² und R³ eine Alkylgruppe mit 1 - 4 C-Atomen oder eine 2-Hydroxyalkylgruppe mit 2 - 4 C-Atomen, R⁴ Wasserstoff, eine Alkylgruppe mit 1 - 4 C-Atomen, eine Benzylgruppe oder eine 2-Hydroxyalkylgruppe mit 2 - 4 C-Atomen, R⁵ eine Alkylengruppe mit 1 - 4 C-Atomen, x = 0 oder 1 und A⁽⁻⁾ ein Fluorid-, Chlorid-, Bromid-, Methoxy- oder Ethoxysulfat-Anion ist, in einer Menge von 0,01 - 1 Gew.-% enthalten ist.

4. Zahnpaste nach einem der Patentansprüche 1 - 3, dadurch gekennzeichnet, daß 0,1 - 0,5 Gew.-% eines Aromaöls und 0,1 - 0,7 Gew.-% eines Lösungsvermittlers aus der Gruppe der oxethylierten Fettsäureglyceride, der oxethylierten Fettsäure-sorbitanpartialester oder der Fettsäurepartialester von Glycerin- oder Sorbitan-Oxethylaten enthalten ist.

5. Zahnpaste nach einem der Patentansprüche 1 - 4, dadurch gekennzeichnet, daß als Lösungsvermittler für das Aromaöl ein Anlagerungsprodukt von 20 - 60 Mol Ethylenoxid an gehärtetes oder ungehärtetes Ricinusöl, an Glycerinmono- und/oder -distearat oder an Sorbitanmono- und/oder -distearat enthalten ist.

## Claims

1. A toothpaste in the form of an aqueous dispersion containing 10.0 to 60.0% by weight polishes, 2.0 to 20.0% by weight humectants, 0.5 to 5.0% by weight water-soluble viscosity regulators, 0.05 to 0.5% by weight antimicrobial biguanides and 1.0 to 5.0% by weight other additives from the group of surfactants, flavoring oils and sweeteners, characterized in that calcium carbonate is predominantly present as the polish, nonionic polysaccharide derivatives are present as the viscosity regulators and a cationic surfactant containing a linear C₁₂₋₁₈ alkyl group and one or two tertiary amino groups or quaternary ammonium groups is present as the surfactant, a nonionic solubilizer for the flavoring oil optionally being present.

2. A toothpaste as claimed in claim 1, characterized in that 1,1'-hexamethylene-bis-(4-chlorophenyl)-biguanide (chlorhexidine) in the form of a water-soluble salt is present as the antimicrobial biguanide.

3. A toothpaste as claimed in claim 1 or 2, characterized in that a cationic surfactant corresponding to formula (I) 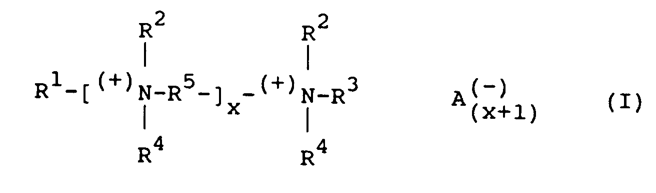 in which R¹ is a C₁₂₋₁₈ alkyl or hydroxyalkyl group, R² and R³ represent a C₁₋₄ alkyl group or a C₂₋₄ 2-hydroxyalkyl group, R⁴ is hydrogen, a C₁₋₄ alkyl group, a benzyl group or a C₂₋₄ 2-hydroxyalxyl group, R⁵ is a C₁₋₄ alkylene group, x = 0 or 1 and A⁽⁻⁾ is a fluoride, chloride, bromide, methoxysulfate or ethoxysulfate anion, is present as the cationic surfactant in a quantity of 0.01 to 1% by weight.

4. A toothpaste as claimed in any of claims 1 to 3, characterized in that it contains 0.1 to 0.50% by weight of a flavoring oil and 0.1 to 0. 7% by weight of a solubilizer from the group of ethoxylated fatty acid glycerides, ethoxylated fatty acid sorbitan partial esters or fatty acid partial esters of glycerol or sorbitan ethoxylates.

5. A toothpaste as claimed in any of claims 1 to 4, characterized in that an adduct of 20 to 60 mol ethylene oxide with hydrogenated or non-hydrogenated castor oil, with glycerol mono- and/or distearate or with sorbitan mono- and/or distearate is present as the solubilizer for the flavoring oil.

## Revendications

1. Dentifrice sous la forme d'une dispersion aqueuse renfermant
10,0 à 60,0 % en poids d'agent polissant,
2,0 à 20,0 % en poids d'agent d'humidification,
0,5 à 5,0 % en poids de régulateurs de consistance solubles dans l'eau,
0,05 à 0,5 % en poids de biguanide antimicrobien et
1,0 à 5,0 % en poids d'autres additifs faisant partie du groupe des substances tensioactives, des huiles aromatiques et des édulcorants, caractérisé en ce que sont contenus
- comme agents polissants, essentiellement du carbonate de calcium
- comme régulateurs de consistance, des dérivés polysaccharidiques non ioniques et
- comme substances tensioactives, un surfactif cationique présentant un groupe alkyle linéaire comportant 12 à 18 atomes de C et un ou deux groupes amino tertiaires ou ammonium quaternaires et, éventuellement, un solubiliseur non ionique pour l'huile aromatique.

2. Dentifrice selon la revendication 1, caractérisé en ce qu'il contient comme biguanide antimicrobien, le 1,1'-hexaméthylène-bis-(4-chlorophényl)-biguanide (chlorhexidine), sous la forme d'un sel soluble dans l'eau.

3. Dentifrice selon une des revendications 1 ou 2, caractérisé en ce qu'il contient comme tensioactif cationique, un surfactif de la formule I 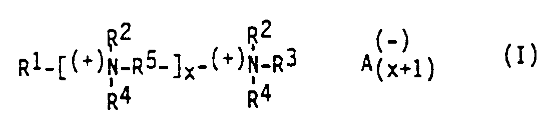 dans laquelle R¹ représente un groupe alkyle ou hydroxyalkyle comportant 12 à 18 atomes de C, R² et R³ correspondent à un groupe alkyle possédant 1 à 4 atomes de C ou à un groupe 2-hydroxyalkyle comportant 2 à 4 atomes de C, R⁴ est l'hydrogène, un groupe alkyle présentant 1 à 4 atomes de C, un groupe benzyle ou un groupe 2-hydroxyalkyle comportant 2 à 4 atomes de C, R⁵ représente un groupe alkylène possédant 1 à 4 atomes de C, x = 0 ou 1 et A⁽⁻⁾ représente un anion fluorure, chlorure, bromure, méthoxy- ou éthoxysulfate, contenu dans une proportion de 0,01 à 1 % en poids.

4. Dentifrice selon une des revendications 1 à 3, caractérisé en ce qu'il contient 0,1 à 0,5 % en poids d'une huile aromatique et 0,1 à 0,7 % en poids d'un solubiliseur, appartenant au groupe constitué des glycérides d'acides gras oxéthylés, des esters partiels de sorbitane d'acides gras oxéthylés ou des esters partiels d'acides gras d'oxéthylates de glycérine ou de sorbitane.

5. Dentifrice selon une des revendications 1 à 4, caractérisé en ce qu'il contient comme solubiliseur pour l'huile aromatique un produit d'addition de 20 à 60 moles d'oxyde d'éthylène à de l'huile de ricin durcie ou non durcie, à un mono- et/ou à un distéarate de glycérine ou à un mono et/ou à un distéarate de sorbitane.
